# EUROPÄISCHE PATENTSCHRIFT

(11) **EP 1 891 059 B1**
(45) Veröffentlichungstag und Bekanntmachung des Hinweises auf die Patenterteilung: **05.12.2012**
(21) Anmeldenummer: 06753478.4
(22) Anmeldetag: 03.05.2006
(51) Int. Cl.: C07D 407/04, C09K 19/34

(54) **PYRAN-DIOXAN-DERIVATE UND DEREN VERWENDUNG IN FLÜSSIGKRISTALLINEN MEDIEN**
PYRAN/DIOXAN DERIVATIVES AND USE THEREOF IN LIQUID CRYSTAL MEDIA
DERIVES DE PYRANE-DIOXANE ET UTILISATION DE CEUX-CI DANS DES MILIEUX A CRISTAUX LIQUIDES

(30) Priorität: 25.05.2005 EP 05011324; 12.08.2005 EP 05017654
(43) Veröffentlichungstag der Anmeldung: 27.02.2008
(73) Patentinhaber: Merck Patent GmbH, 64293 Darmstadt (DE)
(72) Erfinder: LIETZAU, Lars, 64295 Darmstadt (DE); CZANTA, Markus, 64289 Darmstadt (DE)
(86) Internationale Anmeldenummer: PCT/EP2006/004140
(87) Internationale Veröffentlichungsnummer: WO 2006/125511

(56) Entgegenhaltungen:
- EP-A- 0 117 476
- EP-A- 1 362 839
- WO-A-2004/106459
- DE-A1- 10 318 420

## Beschreibung

Die Erfindung betrifft Pyran-Dioxan-Derivate sowie ihre Verwendung als Komponente(n) in flüssigkristallinen Medien. Darüber hinaus betrifft die vorliegende Erfindung Flüssigkristall- und elektrooptische Anzeigeelemente, welche die erfindungsgemäßen, flüssigkristallinen Medien enthalten.

In den vergangenen Jahren wurden die Anwendungsgebiete für flüssigkristalline Verbindungen auf verschiedene Arten von Anzeigevorrichtungen, elektrooptische Geräte, elektronische Komponenten, Sensoren, etc. erheblich ausgeweitet. Aus diesem Grund wurden eine Reihe verschiedener Strukturen vorgeschlagen, insbesondere auf dem Gebiet der nematischen Flüssigkristalle, die bisher die breiteste Anwendung in flüssigkristallinen Anzeigevorrichtungen gefunden haben. Dabei wurden besonders passive TN- oder STN-Matrixanzeigen oder Systeme mit einer TFT-Aktivmatrix eingesetzt.

Die erfindungsgemäßen, flüssigkristallinen Verbindungen können als Komponente(n) flüssigkristalliner Medien verwendet werden, insbesondere für Displays, die auf dem Prinzip der verdrillten Zelle, dem Guest-Host-Effekt, dem Effekt der Deformation aufgerichteter Phasen DAP oder ECB (electrically controlled birefringence), dem IPS-Effekt (in-plane switching) oder dem Effekt der dynamischen Streuung beruhen.

Die Verwendung bestimmter Tetrahydropyran-Derivate als flüssigkristalline Substanzen ist bekannt.

In der DE 102004025808 A1 wird die Synthese einer Verbindung offenbart, die aus 3 Ringen bestehen, wobei je ein Tetrahydropyranring und ein Dioxanring enthalten ist. Die Substanzen besitzen positive Werte der dielektrischen Anisotropie Δε.

Darüber hinaus wurden bereits verschiedene Tetrahydropyran-Derivate als flüssigkristallines Material und deren Herstellung beschrieben, wie z. B. in der DE 102004025809 A1, DE 10318420 A1 oder WO 2004/048357 A1.

Der vorliegenden Erfindung lag die Aufgabe zugrunde, neue stabile flüssigkristalline oder mesogene Verbindungen aufzufinden, die als Komponente(n) flüssigkristalliner Medien geeignet sind. Insbesondere sollen die Verbindungen gleichzeitig eine vergleichsweise geringe Viskosität, sowie eine dielektrische Anisotropie im positiven Bereich besitzen. Für viele aktuelle Mischungskonzepte im Bereich der Flüssigkristalle ist es vorteilhaft, Verbindungen mit einer besonders hohen dielektrischen Anisotropie Δε zu verwenden.

Im Hinblick auf die verschiedensten Einsatzbereiche derartiger Verbindungen mit hohem Δε war es wünschenswert, weitere Verbindungen, vorzugsweise mit hoher Nematogenität zur Verfügung zu haben, die auf die jeweiligen Anwendungen genau maßgeschneiderte Eigenschaften aufweisen.

Der Erfindung lag somit als eine Aufgabe zugrunde, neue stabile, flüssigkristalline oder mesogene Verbindungen aufzufinden, die als Komponente(n) flüssigkristalliner Medien, insbesondere für TN-, STN-, IPS- und TFT-Displays, geeignet sind.

Eine weitere Aufgabe der vorliegenden Erfindung war es, flüssigkristalline oder mesogene Verbindungen bereitzustellen, die für sich oder in Mischungen eine hohe dielektrische Anisotropie Δε, einen hohen Klärpunkt sowie eine niedrige Rotationsviskosität γ₁ aufweisen. Darüber hinaus sollten die erfindungsgemäßen Verbindungen thermisch und photochemisch stabil sein. Ferner sollten die erfindungsgemäßen Verbindungen eine möglichst breite nematische Phase aufweisen. Als Mesogene sollten sie eine breite nematische Phase in Mischungen mit flüssigkristallinen Cokomponenten ermöglichen sowie hervorragend mit nematischen Basismischungen, insbesondere bei tiefen Temperaturen, mischbar sein.

Überraschenderweise wurde gefunden, dass die erfindungsgemäßen Pyran-Dioxan-Derivate vorzüglich als Komponenten flüssigkristalliner Medien geeignet sind. Mit ihrer Hilfe lassen sich stabile, flüssigkristalline Medien, insbesondere geeignet für TN-TFT- und STN-Displays, aber auch für IPS-Systeme oder neuere Konzepte, die besonders hohe dielektrische Anisotropien benötigen, erhalten. Die erfindungsgemäßen Verbindungen sind sowohl thermisch als auch UV-stabil. Auch zeichnen sie sich durch stark positive dielektrische Anisotropien Δε aus, aufgrund derer in der Anwendung in optischen Schaltelementen niedrigere Schwellenspannungen erforderlich sind. Besonders hohe Werte erreicht insbesondere auch der Quotient Δε/Δn der erfindungsgemäßen Verbindungen, d. h. bei gleichen Werten von Δε werden durch die erfindungsgemäßen Substanzen relativ kleine Werte der optischen Anisotropie Δn ermöglicht. Darüber hinaus weisen die erfindungsgemäßen Verbindungen einen hohen Klärpunkt sowie gleichzeitig günstige Werte für die Rotationsviskosität auf.

Mit der Bereitstellung der erfindungsgemäßen Pyran-Dioxan-Derivate wird ganz allgemein die Palette der flüssigkristallinen Substanzen, die sich unter verschiedenen, anwendungstechnischen Gesichtspunkten zur Herstellung flüssigkristalliner Mischungen eignen, erheblich verbreitert.

Die erfindungsgemäßen Pyran-Dioxan-Derivate besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind. Es können aber auch den erfindungsgemäßen Verbindungen flüssigkristalline Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die erfindungsgemäßen Pyran-Dioxan-Derivate sind in reinem Zustand farblos. Thermisch und gegen Licht sind sie stabil.

Gegenstand der vorliegenden Erfindung sind somit Pyran-Dioxan-Derivate der allgemeinen Formel I 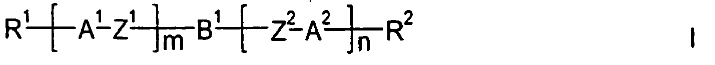 worin
- B¹: 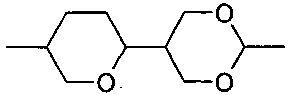
- R¹, R²: H, Halogen, CN, SCN, NCS, SF₅, einen linearen oder verzweigten, gegebenenfalls chiralen, unsubstituierten, einen einfach durch CN oder CF₃ substituierten oder einen ein- oder mehrfach durch Halogen substituierten Alkylrest mit 1 bis 15 C-Atomen bedeutet, worin auch eine oder mehrere CH₂-Gruppen jeweils unabhängig voneinander durch -O-, -S-, -CO-, -CO-O-, -O-CO-, -CH=CH-, -CH=CF-, -CF=CF-, -C≡C-, -CH₂O- oder -CF₂O-so ersetzt sein können, dass Heteroatome nicht direkt miteinander verknüpft sind und unsymmetrische Gruppen in beiden Orientierungen vorliegen können,
- A¹, A²: jeweils unabhängig voneinander, gleich oder verschieden
a) trans-1,4-Cyclohexylen, worin auch eine oder mehrere nicht benachbarte CH₂-Gruppen durch -O- und/oder -S-ersetzt sein können,
b) 1,4-Phenylen, worin eine oder zwei CH-Gruppen durch N ersetzt sein können und worin auch ein oder mehrere H-Atome gegen Br, Cl, F, CN, Methyl, Methoxy oder eine ein- oder mehrfach fluorierte Methyl- oder Methoxygruppe ersetzt sein können,
c) ein Rest aus der Gruppe 1,4-Bicyclo(2,2,2)-octylen, Piperidin-1,4-diyl, Cylcobut-1,3-diyl, Spiro[3.3]heptan-2,6-diyl, 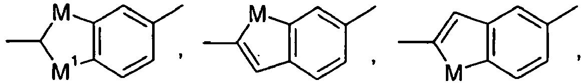 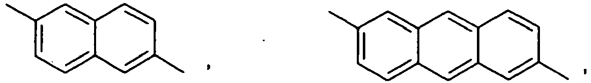 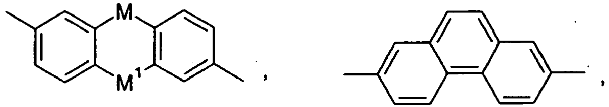 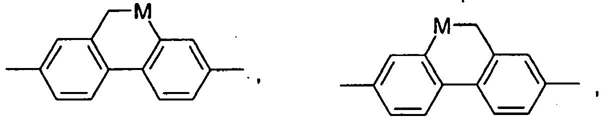 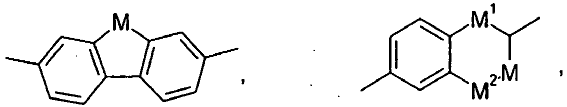 worin Wasserstoffatome ein oder mehrfach durch F, CN, SCN, SF₅, CH₂F, CHF₂ oder CF₃, OCH₂F, OCHF₂ oder OCF₃ substituiert sein können, eine oder mehrere Doppelbindungen durch Einfachbindungen ersetzt sein können, M, M¹ oder M² -O-, -S-, -CH₂-, -CHY- oder -CYY¹-, und Y und Y¹ Cl, F, CN, OCF₃ oder CF₃ bedeuten, oder
d) 1,4-Cyclohexenylen,
- Z¹, Z²: jeweils unabhängig voneinander, gleich oder verschieden, eine Einfachbindung, -CH₂O-, -CO-O-, -CF₂O-, -CH₂CH₂CF₂O-, -CF₂CF₂-, -CH₂CF₂-, -CH₂CH₂-, -CH=CH-, -CH=CF-, -CF=CF- oder -C≡C-, wobei asymmetrische Brücken nach beiden Seiten orientiert sein können, und
- n, m: unabhängig voneinander 0, 1, 2 oder 3 bedeuten, wobei m+n ≥2 ist.

Ein weiterer Gegenstand der vorliegenden Erfindung ist die Verwendung von Verbindungen der Formel I als Komponente(n) in flüssigkristallinen Medien.

Ebenfalls Gegenstand der vorliegenden Erfindung sind flüssigkristalline Medien mit mindestens zwei flüssigkristallinen Komponenten, welche mindestens ein Pyran-Dioxan-Derivat der Formel I enthalten.

Gegenstand der vorliegenden Erfindung sind auch Flüssigkristall-Anzeigeelemente, insbesondere elektrooptische Anzeigeelemente, welche als Dielektrikum ein erfindungsgemäßes, flüssigkristallines Medium enthalten.

Die Bedeutung der Formel I schließt alle Isotope der in den Verbindungen der Formel I gebundenen chemischen Elemente ein. In enantiomerenreiner oder -angereicherter Form eignen sich die Verbindungen der Formel I prinzipiell auch als chirale Dotierstoffe und generell zur Erzielung chiraler Mesophasen.

Vor- und nachstehend haben R¹, R², A¹, A², Z¹, Z², n und m die angegebenen Bedeutungen, sofern nicht ausdrücklich etwas anderes vermerkt ist. Kommen die Reste A¹, A², Z¹ und Z² durch umgebende Klammern mehrfach vor, so können sie unabhängig voneinander, gleiche oder verschiedene Bedeutungen annehmen.
Ist der Ring A¹ oder A² zweimal vorhanden, so können die beiden Ringe gleiche oder verschiedene Bedeutungen haben. Entsprechendes gilt für 3 Ringe. Dasselbe gilt auch für die Brücken Z¹ und Z².

Bevorzugte Ausführungsformen der erfindungsgemäßen Verbindungen der allgemeinen Formel I sind im Folgenden beschrieben:

In einer bevorzugten Ausführungsform sind die Verbindungen der Formel I dadurch gekennzeichnet, dass n + m 3 ist.

Dabei sind Verbindungen der Formel I besonders bevorzugt, in denen
n gleich 2 und
m gleich 0 oder 1 ist.

Bevorzugt sind ebenso Verbindungen der Formel I, die dadurch gekennzeichnet sind, dass
n 1, 2 oder 3 bedeutet, und
mindestens ein Z²
-CF₂O-, -CO-O-, -CF=CF-, -CH₂O- oder -CF₂CF₂-
bedeutet.

Unter den möglichen Ringen A¹ und A² in Formel I sind Ringe gemäß Definition a) und b), sowie ferner Ringe der Gruppen d) und carbocyclische Zweiringsysteme aus c) wie z. B. Indane bevorzugt.

Unter den möglichen Gruppen Z¹ und Z² sind die Gruppen -CF₂O-, -OCF₂-, -CF₂CF₂-, -CF=CF- oder die Einfachbindung bevorzugt. Ganz besonders bevorzugt ist die Einfachbindung.

Bevorzugt sind Verbindungen der Formel IA 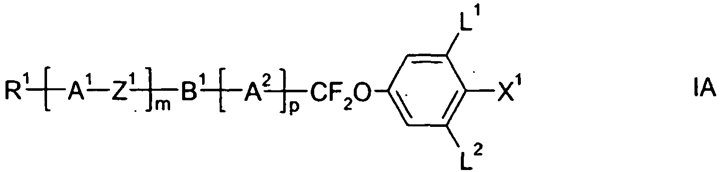 worin,
- L¹, L²: unabhängig voneinander, H, F, Cl, CN oder CF₃,
- p: 0, 1 oder 2, bevorzugt 0 oder 1,
- m + p: 0, 1, oder 2, bevorzugt 0 oder 1, und
- X¹: H, Halogen, CN, SCN, NCS, SF₅, einen linearen oder verzweigten, einfach durch CN oder CF₃ oder einen mindestens einfach durch Halogen substituierten Alkylrest mit 1 bis 8 C-Atomen bedeutet, worin auch eine oder mehrere CH₂-Gruppen jeweils unabhängig voneinander durch -O-, -CF=CF- oder -C≡C- so ersetzt sein können, dass Heteroatome nicht direkt miteinander verknüpft sind, also auch z. B. OCF₃ oder -CF=CF₂, bedeuten, und die übrigen Variablen wie für Formel I oben definiert sind.

In den Formeln I und IA bedeutet Z¹ vorzugsweise -CH₂CH₂-, -CH=CH-, -C≡C-, -CF₂CF₂-, -CF=CF-, -CO-O-, -O-CO-, -CF₂O-, -OCF₂- oder eine Einfachbindung, besonders bevorzugt -CF₂O-, -CF₂CF₂- oder eine Einfachbindung. Für den Fall, dass m > 1 ist, bedeutet vorzugsweise mindestens eines der Z¹ eine Einfachbindung.

Ganz besonders bevorzugt sind Verbindungen der Formel IB 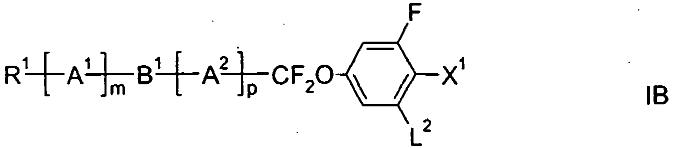 worin
- L²: H oder F,
- X¹: F oder OCF₃, CF₃, CN, NCS, SCN, SF₅,
- A¹: ein 1,4-Cyclohexandiyl,
- A²: eine Gruppe der Formeln 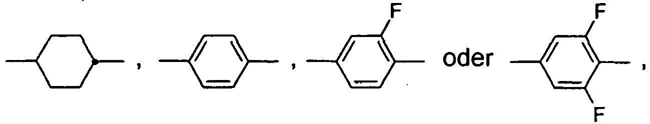
- m: 0 oder 1,
- p: 0 oder 1, bevorzugt 1, und
- m + p: 0 oder 1, bevorzugt 1
bedeuten.

Bevorzugt sind jeweils Verbindungen der Formel I, IA und IB worin R¹ einen linearen Alkyl- bzw. Alkoxyrest mit 1 bis 12 C-Atomen oder einen linearen Alkenyl- bzw. Alkenyloxyrest mit 2 bis 12 C-Atomen bedeutet.

Besonders bevorzugt sind auch die Verbindungen der Formeln IA und IB, worin L² für Fluor steht. Besonders bevorzugt sind auch die Verbindungen der Formeln IA und IB, worin X¹ für Fluor oder OCF₃ steht.

Die ganz besonders bevorzugten erfindungsgemäßen Verbindungen sind demnach I-1 oder 1-3 : 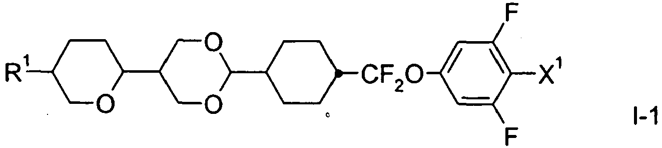 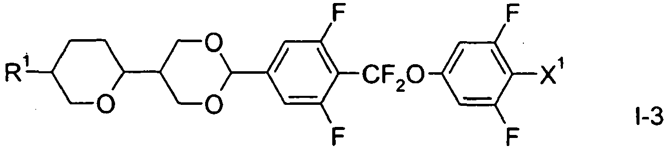 wobei X¹ und R¹ wie für IB definiert sind, und wobei bevorzugt R¹ einen linearen Alkyl- bzw. Alkoxyrest mit 1 bis 10 C-Atomen oder einen linearen Alkenyl- bzw. Alkenyloxyrest mit 2 bis 10 C-Atomen bedeutet. X¹ bedeutet bevorzugt F oder OCF₃.

In den vorstehenden, bevorzugten Formeln bedeutet R¹ vorzugsweise einen linearen Alkyl- bzw. Alkoxyrest mit 1 bis 7 C-Atomen oder einen linearen Alkenyl- bzw. Alkenyloxyrest mit 2 bis 7 C-Atomen in denen eine -CH₂-Gruppe durch -O- so ersetzt sein kann, so dass nicht zwei benachbarte Gruppen durch -O- ersetzt werden. Besonders bevorzugt bedeutet R¹ einen linearen Alkylrest oder Alkoxyrest mit 1 bis 7 C-Atomen oder einen linearen Alkenylrest mit 2 bis 7 C-Atomen.

Falls R¹ oder R² in den vor- und nachstehenden Formeln einen Alkylrest bedeutet, so kann dieser geradkettig oder verzweigt sein. Besonders bevorzugt ist er geradkettig, hat 1, 2, 3, 4, 5, 6 oder 7 C-Atome und bedeutet demnach Methyl, Ethyl, Propyl, Butyl, Pentyl, Hexyl oder Heptyl, ferner Octyl, Nonyl, Decyl, Undecyl, Dodecyl, Tridecyl, Tetradecyl oder Pentadecyl.

Falls R¹ oder R² einen Alkylrest bedeutet, in dem eine CH₂-Gruppe durch - O- ersetzt ist, so kann dieser geradkettig oder verzweigt sein. Vorzugsweise ist er geradkettig und hat 1 bis 10 C-Atome. Besonders bevorzugt ist die erste CH₂-Gruppe dieses Alkylrestes durch -O- ersetzt, so dass der Rest R¹ die Bedeutung Alkoxy erhält und Methoxy, Ethoxy, Propoxy, Butoxy, Pentyloxy, Hexyloxy, Heptyloxy, Octyloxy oder Nonyloxy bedeutet.

Weiterhin kann auch eine CH₂-Gruppe an anderer Stelle durch -O- ersetzt sein, so dass der Rest R¹ und/oder R² vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl bedeutet.

Falls R¹ oder R² einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Bevorzugte Alkenylgruppen sind C₂-C₇₋₁ E-Alkenyl, C₄-C₇-3E-Alkenyl, C₅-C₇-4-Alkenyl, C₆-C₇-5-Alkenyl, und C₇-6-Alkenyl, besonders bevorzugt C₂-C₇-1E-Alkenyl, C₄-C₇-3E-Alkenyl und C₅-C₇-4-Alkenyl.

Beispiele besonders bevorzugter Alkenylgruppen sind Vinyl, 1 E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl und 6-Heptenyl. Gruppen mit bis zu 5 Kohlenstoffatomen sind insbesondere bevorzugt.

Falls R¹ oder R² einen Alkylrest bedeutet, in dem eine CH₂-Gruppe durch -O- und eine durch -CO- ersetzt ist, so sind diese vorzugsweise benachbart. Somit beinhalten diese eine Acyloxygruppe -CO-O- oder eine Oxycarbonylgruppe -O-CO-. Besonders bevorzugt sind diese geradkettig und haben 2 bis 6 C-Atome.

Sie bedeuten demnach insbesondere Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl und 4-(Methoxycarbonyl)butyl.

Falls R¹ oder R² einen Alkylrest bedeutet, in dem eine CH₂-Gruppe durch unsubstituiertes oder substituiertes -CH=CH- und eine benachbarte CH₂-Gruppe durch -CO-, -CO-O- oder -O-CO- ersetzt ist, so kann dieser geradkettig oder verzweigt sein. Vorzugsweise ist er geradkettig und hat 4 bis 13 C-Atome. Er bedeutet demnach besonders bevorzugt Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryfoyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl und 9-Methacryloyloxynonyl.

Falls R' oder R² einen einfach durch CN oder CF₃ substituierten Alkyl- oder Alkenylrest bedeutet, so ist dieser Rest vorzugsweise geradkettig und die Substitution durch CN oder CF₃ in ω-Position.

Falls R' oder R² einen mindestens einfach durch Halogen substituierten Alkyl- oder Alkenylrest bedeutet, so ist dieser Rest vorzugsweise geradkettig. Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I mitt verzweigter Flügelgruppe R¹ können gelegentlich wegen einer besseren Löslichkeit in den üblichen, flüssigkristallinen Basismaterialien von Bedeutung sein, insbesondere aber als chirale Dotierstoffe, wenn sie optisch aktiv sind. Smektische Verbindungen dieser Art eignen sich als Komponente(n) für ferroelektrische Materialien.

Verzweigte Gruppen dieser Art enthalten vorzugsweise nicht mehr als eine Kettenverzweigung. Bevorzugte verzweigte Reste R¹ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentyloxy, 3-Methylpentyloxy, 2-Ethylhexyloxy, 1-Methylhexyloxy und 1-Methylheptyloxy.

Für bestimmte Anwendungen der erfindungsgemäßen Verbindungen, z. B. zur Erzielung besonders niedriger Viskositäten, ist es vorteilhaft, dass die Reihenfolge der Ringe im Strukturelement B¹ Pyran-Dioxan (entsprechend den Abbildungen) bedeutet. Dabei ist Position 2 des Tetrahydropyranringes mit Position 5 des Dioxanringes verknüpft.

Die Formel I und die Unterformeln IA, IB und IC umfassen, falls es sich um chirale Verbindungen handelt, üblicherweise die Racemate dieser Verbindungen, aber auch beide optisch reinen Komponenten für sich, sowie angereicherte Gemische dieser Komponenten.

Die Verbindungen der Formel I werden nach an sich bekannten Methoden dargestellt, wie sie in der Literatur (z. B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Der Dioxanring in den Verbindungen wird in der Regel durch Kondensation aus einem Aldehyd mit einem 2-substituierten 1,3-Diol hergestellt. Der Tetrahydropyranring, der dabei entweder am Diol oder an der Aldehydgruppe hängt, wird nach einem der zahlreichen bekannten Verfahren für die Synthese von 2,5-disubstituierten Tetrahydropyranen hergestellt. Für die Tetrahydropyran-2-aldehyde gibt es mehrere Synthesewege: Durch Reduktion eines entsprechenden Carbonsäurederivats, durch milde Oxidation eines entsprechenden Alkohols (Carbinol) oder durch katalytische Hydroformylierung eines geeigneten Dihydropyrans.

Die Ausgangsmaterialien für das obige Verfahren sind entweder bekannt oder können in Analogie zu bekannten Verbindungen hergestellt werden.

Gegebenenfalls können die Ausgangsstoffe auch *in situ* gebildet werden, derart, dass man sie aus dem Reaktionsgemisch nicht isoliert, sondern sofort weiter zu den Verbindungen der Formel I umsetzt.

Die dargestellten Reaktionen sind nur als beispielhaft aufzufassen. Der Fachmann kann entsprechende Varianten der vorgestellten Synthesen vornehmen sowie auch andere geeignete Synthesewege beschreiten, um die erfindungsgemäßen Verbindungen der Formel I zu erhalten.

Die Synthesen verschiedener Derivate der allgemeinen Formel I werden außerdem in den Beispielen ausführlich beschrieben. Die Synthesemethoden lassen sich variieren, so dass mit Hilfe von abgeänderten Edukten alle erfindungsgemäßen Verbindungen dargestellt werden können.

Die erfindungsgemäßen, flüssigkristallinen Medien enthalten vorzugsweise neben einer oder mehreren erfindungsgemäßen Verbindungen als weitere Bestandteile 2 bis 40, besonders bevorzugt 4 bis 30 Komponenten. Insbesondere enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile der erfindungsgemäßen Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Py-, -G-Phe- und -G-Cyc- sowie deren Spiegelbildern gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl, Py Tetrahydropyran-2,5-diyl- und G 2-(trans-1,4-Cyclohexyl)-ethyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe, Py und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und/oder R" bedeuten jeweils unabhängig voneinander Alkyl, Alkenyl, Alkoxy, Alkoxyalkyl, Alkenyloxy oder Alkanoyloxy mit bis zu 8 C-Atomen, -F, -Cl, -CN, -NCS, -(O)ᵢCH₃₋₍ₖ₊ₗ₎FₖClₗ, wobei i 0 oder 1, k und l unabhängig voneinander, gleich oder verschieden, 0, 1, 2 oder 3 sind, jedoch mit der Maßgabe, dass die Summe (k + l) 1, 2 oder 3 ist.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 C-Atomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢ CH₃₋₍ₖ₊₁₎ FₖClₗ, wobei i 0 oder 1, k und l unabhängig voneinander 0, 1, 2 oder 3 sind, jedoch mit der Maßgabe, dass die Summe (k + l) 1, 2 oder 3 ist. Die Verbindungen, in denen R" diese Bedeutung hat, werden mit den Teilformeln 1b, 2b, 3b, 4b und 5b bezeichnet. Besonders bevorzugt sind solche Verbindungen der Teilformeln 1 b, 2b, 3b, 4b und 5b, in denen R" die Bedeutung -F, -Cl, -NCS, -CF₃, -OCHF₂ oder -OCF₃ hat.

In den Verbindungen der Teilformeln 1b, 2b, 3b, 4b und 5b hat R' die bei den Verbindungen der Teilformeln 1a bis 5a angegebenen Bedeutungen und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN. Diese Untergruppe wird im folgenden als Gruppe C bezeichnet und die Verbindungen dieser Untergruppe werden entsprechend mit Teilformeln 1 c, 2c, 3c, 4c und 5c beschrieben. In den Verbindungen der Teilformeln 1 c, 2c, 3c, 4c und 5c hat R' die bei den Verbindungen der Teilformeln 1a bis 5a angegebenen Bedeutungen und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus den Gruppen A, B und/oder C. Die Massenanteile der Verbindungen aus diesen Gruppen in den erfindungsgemäßen Medien sind vorzugsweise:
- Gruppe A:: 0 bis 90 %, vorzugsweise 20 bis 90 %, besonders bevorzugt 30 bis 90 %;
- Gruppe B:: 0 bis 80 %, vorzugsweise 10 bis 80 %, besonders bevorzugt 10 bis 65 %;
- Gruppe C:: 0 bis 80 %, vorzugsweise 5 bis 80 %, besonders bevorzugt 5 bis 50 %;
wobei die Summe der Massenanteile der in den jeweiligen erfindungsgemäßen Medien enthaltenen Verbindungen aus den Gruppen A, B und/oder C vorzugsweise 5 bis 90 % und besonders bevorzugt 10 bis 90 % beträgt.

Die erfindungsgemäßen Medien enthalten vorzugsweise 1 bis 40 %, besonders bevorzugt 5 bis 30 %, der erfindungsgemäßen Verbindungen. Des weiteren bevorzugt sind Medien, enthaltend mehr als 40 %, besonders bevorzugt 45 bis 90 %, an erfindungsgemäßen Verbindungen. Die Medien enthalten vorzugsweise drei, vier oder fünf erfindungsgemäße Verbindungen.

Die Herstellung der erfindungsgemäßen Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, vorzugsweise bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Weiterhin ist es möglich, die Mischungen auf andere herkömmliche Arten, z. B. durch Verwendung von Vormischungen, z.B. Homologen-Mischungen oder unter Verwendung von sogenannten "Multi-Bottle"-Systemen herzustellen.

Die Dielektrika können auch weitere, dem Fachmann bekannte und in der Literatur beschriebene Zusätze enthalten. Beispielsweise können 0 bis 15 %, vorzugsweise 0 bis 10 %, pleochroitische Farbstoffe und/oder chirale Dotierstoffe zugesetzt werden. Die einzelnen, zugesetzten Verbindungen werden in Konzentrationen von 0,01 bis 6 %, vorzugsweise von 0,1 bis 3 %, eingesetzt. Dabei werden jedoch die Konzentrationsangaben der übrigen Bestandteile der Flüssigkristallmischungen also der flüssigkristallinen oder mesogenen Verbindungen, ohne Berücksichtigung der Konzentration dieser Zusatzstoffe angegeben.

Die folgenden Beispiele erläutern die Erfindung, ohne sie beschränken zu sollen.

Vor- und nachstehend werden folgende Abkürzungen verwendet:
- RT: Raumtemperatur
- THF: Tetrahydrofuran
- MTB-Ether: Methyl-*tert*-butylether
- LAH: Lithimaluminiumhydrid
- p-TsOH: *p*-Toluolsulfonsäure
- DC: Dünnschichtchromatographie
- DBN: 1,5-Diazabicyclo[4.3.0]non-5-en
- DMSO: Dimethylsulfoxid
- DMF: Dimethylformamid
- BuLi: n-Buthyllithium

Daneben werden folgende Abkürzungen verwendet:
K: Kristalline Phase; N: Nematische Phase; I: Isotrope Phase, Sm: smektische Phase. Die Zahlen zwischen den Abkürzungen für die Phasen entsprechen den Übergangtemperaturen für den Reinstoff. Die erste Übergangstemperatur ausgehend von der kristallinen Phase (K) entspricht dem Schmelzpunkt.
Temperaturangaben sind, soweit nichts anderes angegeben, in °C.

Außerdem bedeuten Klp. Klärpunkt und γ₁ Rotationsviskosität. Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties of Liquid Crystals - Description of the Measurements Methods", 1998, Merck KGaA, Darmstadt.
Die dielektrische Anisotropie Δε wird bei 1 kHz bestimmt. Die optische Anisotropie Δn wird bei einer Wellenlänge von 589,3 nm bestimmt. Alle Messwerte werden, soweit nicht anders angegeben, bei einer Temperatur von 20 °C ermittelt. Für Klp., Δε, Δn und γ₁ werden 10 Gew.% der zu untersuchenden Substanz in der dielektrisch positiven Mischung ZLI-4792 (Merck KGaA) gelöst gemessen und der Messwert auf 100 % Gehalt extrapoliert.

### Beispiel 1

### Beispiel 1.a

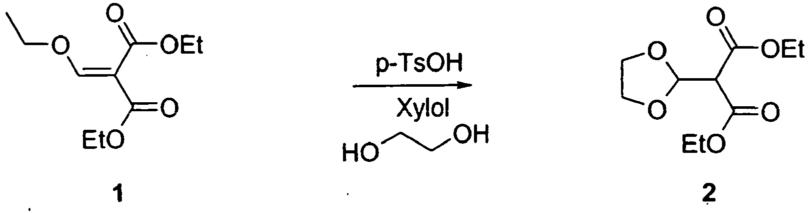

375 ml (1,87 mol) des Malonats **1** wird mit 142 ml (2,55 mol) Ethylenglykol und 9,75 g (50 mmol) p-Toluolsulfonsäure-Monohydrat in 2,1 l Xylol gelöst und zum Sieden erhitzt. Dabei wird bis zu einer Kopftemperatur von 140 °C 1 l Xylol abdestilliert. Das im Kolben verbleibende Gemisch wird mit Natriumhydrogencarbonat gewaschen und eingeengt. Der erhaltene Rückstand bestehend aus **2** wird im Vakuum fraktionierend destilliert.
Ausbeute: 280 g einer farblosen Flüssigkeit.

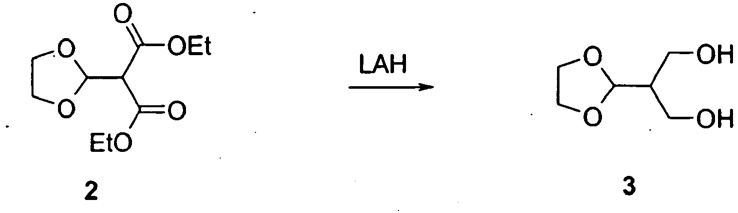

Unter Stickstoff wird eine Suspension von 45,5 g (1,03 mol) Lithiumaluminiumhydrid in 1 l THF in der Siedehitze mit einer Lösung von 187,2 g (790 mmol) des Malonats **2** in THF versetzt und 1 h zum Sieden erhitzt. Der abgekühlte Ansatz wird mit einem THF/Wasser-Gemisch (4:1) hydrolysiert und mit einer 80 °C warmen Lösung von Natriumcarbonat-Decahydrat in 123 ml Wasser versetzt. Nach 230 min wird der entstandene Feststoff **3** abgetrennt und mit MTB-Ether gewaschen. Die organische Phase wird eingeengt und ohne weitere Reinigung in der folgenden Stufe eingesetzt.

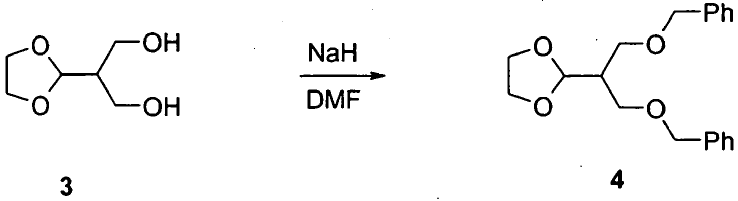

Unter Stickstoff werden 86,3 g (580 mmol) des Diols **3** in 1100 ml DMF gelöst und mit 14 g (38 mmol) Tetra-n-butylammoniumiodid versetzt. Anschließend werden 87,2 g (2,18 mol) einer 60 %igen Natriumhydridsuspension in Mineralöl portionsweise eingetragen. Nach 30 min bei RT werden vorsichtig 264 ml (2,18 mol) Benzylbromid unter Kühlung zugegeben. Nach 48 h bei RT wird der Ansatz auf 3 l Wasser gegeben und mit MTB-Ether extrahiert. Die organische Phase wird mit Wasser gewaschen und eingeengt. Der Rückstand wird über Kieselgel gegeben (Toluol). Es werden 3 Fraktionen von **4** isoliert.

| | |
|---|---|
| 69,6 g; | Gehalt 62,9 % |
| 194,1 g; | Gehalt 92,1 % |
| 23,0 g; | Gehalt 64,8 % |

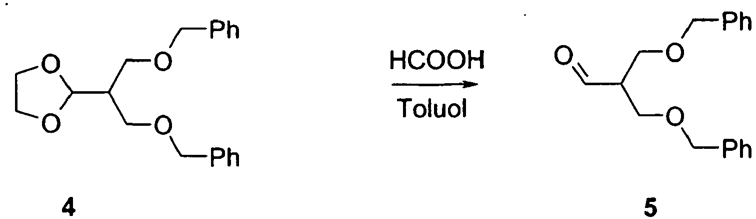

Eine Lösung von 194 g (92,1 %; 540 mmol) des Acetals **4** in 830 ml Toluol wird mit 290 ml Ameisensäure versetzt und unter starkem Rühren 6 h bei 60 °C gehalten. Der abgekühlte Ansatz wird mit 1 l Heptan und 1 l Wasser versetzt. Die organische Phase wird mit Wasser und Natriumhydrogencarbonat-Lösung gewaschen und eingeengt. Der Rückstand wird über Kieselgel gegeben (Toluol/Heptan). Es werden 163,7 g des Aldehyds **5** isoliert.

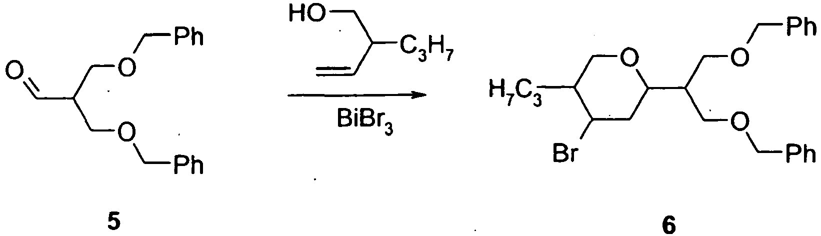

22,5 g (63 %ig; 50 mmol) des Aldehyds **5** und 5,95 g (96 %ig; 50 mmol) 2-Vinyl-propanol werden in 140 ml Dichlormethan gelöst und mit 11,4 g (25 mmol) Bismut(III)bromid versetzt. Der Ansatz wird über Nacht bei RT gerührt. Anschließend wird der Ansatz über Kieselgel filtriert und eingeengt. Es werden 26,1 g der Bromverbindung **6** isoliert.

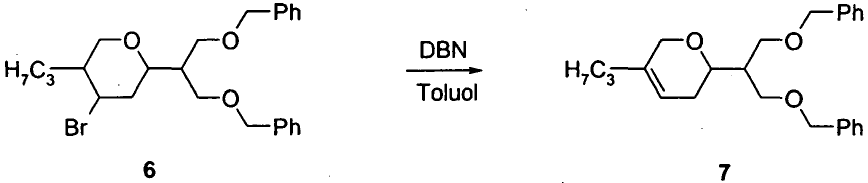

Unter Stickstoff werden 100 g (219 mmol) der Bromverbindung **6** in 165 ml Toluol gelöst, mit 38,5 ml DBN versetzt und 5 h zum Sieden erhitzt. Anschließend wird der abgekühlte Ansatz mit 200 ml Wasser versetzt und mit verdünnter Schwefelsäure angesäuert. Die organische Phase wird mit 300 ml Heptan verdünnt, abgetrennt, mit Natriumhydrogencarbonat-Lösung gewaschen und eingeengt. Der erhaltene Rückstand wird über Kieselgel gegeben (Toluol). Es werden 57,1 g der Verbindung **7** isoliert.

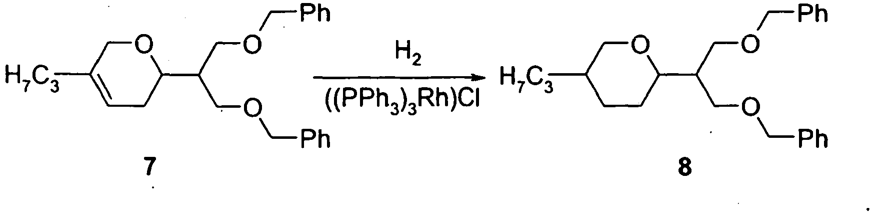

57 g (150 mmol) des ungesättigten Pyrans **7** werden in 360 ml Methanol und 90 ml Toluol gelöst und am (PPh₃)₃RhCl-Katalysator bei 8 bar / 80 °C hydriert. Die Hydrierlösung wird eingeengt und der Rückstand über Kieselgel gegeben (Toluol/MTB-Ether). Es werden zwei Fraktionen des Pyrans 8 erhalten: 32,1 g und 21,6 g .

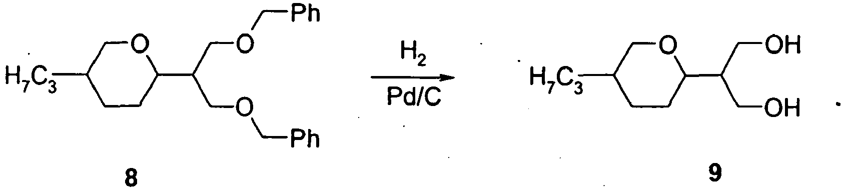

32,1 g (77 %ig) des geschützten Diols **8** werden in 321 ml THF gelöst und am Palladium-Katalysator hydriert. Anschließend wird der Katalysator abgetrennt und die Lösung eingeengt. Der erhaltene Rückstand aus **9** wird ohne weitere Reinigung in der folgenden Stufe eingesetzt.

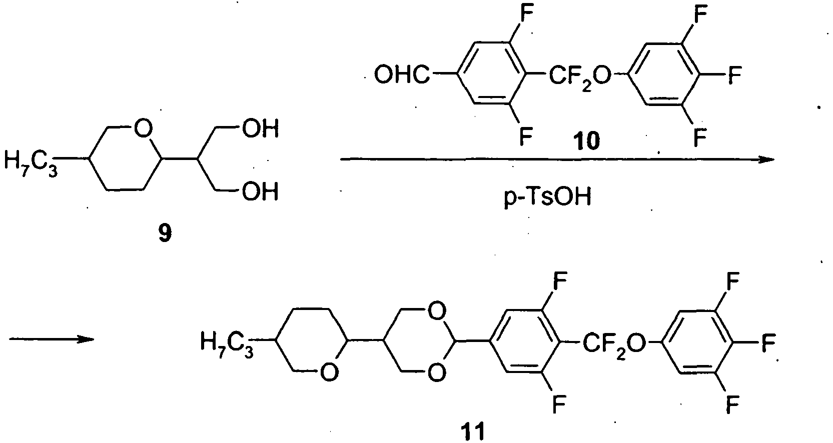

15,8 g (78 mmol) des Diols **9** werden mit 27,4 g (78 mmol) des Aldehyds **10** in 100 ml Toluol gelöst, mit 500 mg p-Toluolsulfonsäure-Monohydrat versetzt und am Wasserabscheider zum Sieden erhitzt.

Anschließend wird der Ansatz über Kieselgel gegeben und das Eluat eingeengt. Der erhaltene Rückstand aus **11** wird durch Kristallisation aus Acetonitril, Aceton und Heptan gereinigt.

### Beispiel 1.b

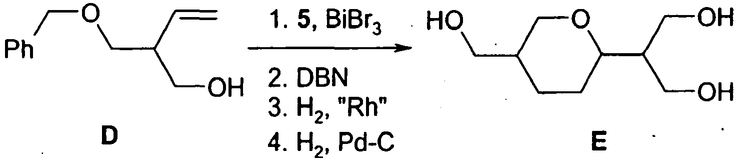

Eine zu Beispiel 1.a analoge Verbindung mit Doppelbindung in der endständigen Seitenkette kann über den Homoallylalkohol **D** hergestellt werden, der nach J.N. Zonjee et al., Tetrahedron 1989, 45, 7553-7567 hergestellt wird. Dazu wird die Verbindung **D** analog Beispiel 1.a nacheinander mit **5** zum Brompyran, dann mit DBU in Toluol und anschließender Hydrierung (nach Abspaltung aller Schutzgruppen, siehe Reaktion **8** -> **9**) zu Verbindung **E** umgesetzt.

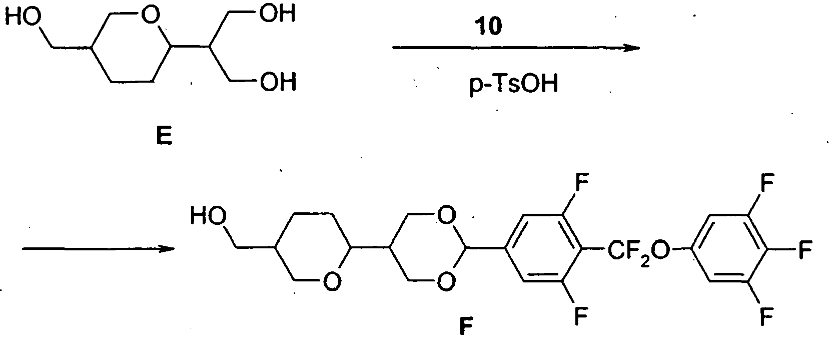

Der Ringschluss der Trihydroxyverbindung **E** zum Dioxan **F** erfolgt durch Kondensation mit dem Aldehyd **10** in Analogie zur Reaktion **9** -> **11.**

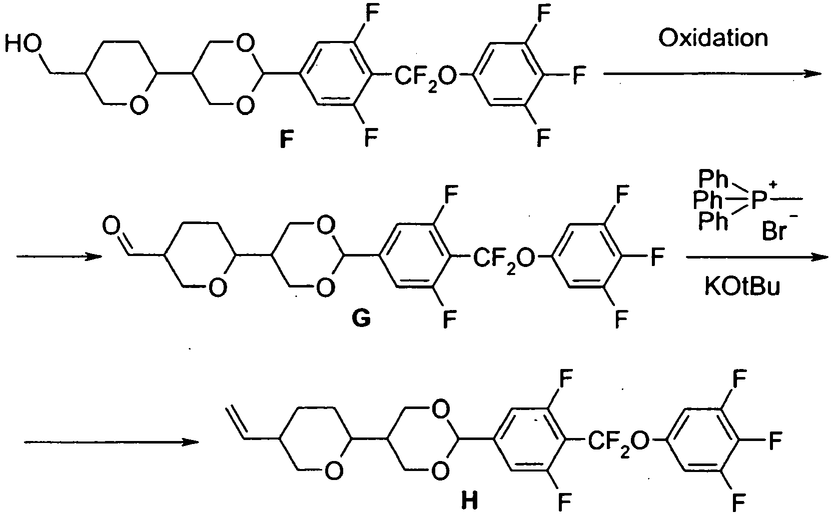

In der weiteren Synthese wird die verbliebene OH-Funktion an Formel **F** nach Swem, mit NaOAc/PCC oder nach Dess-Martin mit Periodinan zum Aldehyd oxidiert und über eine Wittig-Reaktion in eine Doppelbindung übergeführt (**H**). Je nach Wittig-Salz kann die Position der Doppelbindung variiert werden (über mehrere Reaktionsschritte), oder zweifach substituiert sein. Auch die Einführung von mehreren Doppelbindungen ist möglich.

Aus jeweils einem Diol analog Verbindung **9** und einem passenden Aldehyd analog **10** werden die folgenden Verbindungen hergestellt: 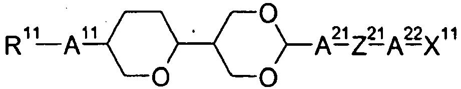 wobei R¹¹, A¹¹, A²¹, Z²¹, A²² und X¹¹ insbesondere gemäß Tabelle 1 bedeuten:

**Tabelle 1: Verbindungen zu Beispiel 1.**

| **#** | **R¹¹** | **A¹¹** | **A²¹** | **Z²¹** | **A²²** | **X¹¹** | **Werte** |
|---|---|---|---|---|---|---|---|
| 1 | CH₃ | - | 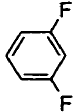 | -CF₂O- | 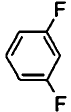 | F | |
| 2 | C₂H₅ | - | 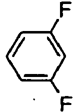 | -CF₂O- | 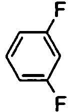 | F | K 93 N (84) I; |
| | | | | | | | Clp. = 79 |
| | | | | | | | Δε = 33,5 |
| | | | | | | | Δn = 0,091 |
| | | | | | | | γ1 = 192 |
| 3 | C₃H₇ | - | 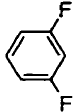 | -CF₂O- | 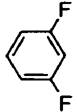 | F | K 92 N 113 I; |
| | | | | | | | Clp. = 97 |
| | | | | | | | Δε = 35 |
| | | | | | | | Δn = 0,099 |
| | | | | | | | γ₁ = 320 |
| 4 | C₄H₉ | - | 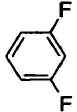 | -CF₂O- | 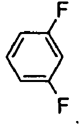 | F | K 88 N 108 I; |
| | | | | | | | Clp. = 97 |
| | | | | | | | Δε = 31 |
| | | | | | | | Δn = 0,093 |
| 5 | C₅H₁₁ | - | 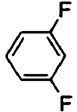 | -CF₂O- | 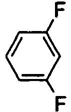 | F | K 86 N 112 I; |
| | | | | | | | Clp. = 104 |
| | | | | | | | Δε = 31 |
| | | | | | | | Δn = 0,094 |
| | | | | | | | γ₁ = 354 |
| 6 | CH₃ | - | 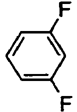 | -CF₂O- | 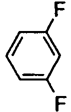 | OCF₃ | |
| 7 | C₂H₅ | - | 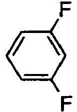 | -CF₂O- | 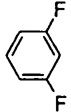 | OCF₃ | K 107 SmA (89) N (120) I |
| | | | | | | | Δε = 34 |
| | | | | | | | Δn = 0,097 |
| 8 | C₃H₇ | - | 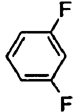 | -CF₂O- | 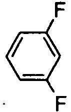 | OCF₃ | K 101 SmA 108 N 135 I; |
| | | | | | | | Δε = 34 |
| | | | | | | | Δn = 0,098 |
| 9 | C₄H₉ | - | 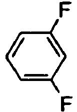 | -CF₂O- | 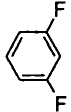 | OCF₃ | K 103 SmA 112 N 132 I; |
| | | | | | | | Clp. = 103 |
| | | | | | | | Δε = 34 |
| | | | | | | | Δn = 0,091 |
| 10 | C₅H₁₁ | - | 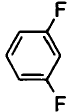 | -CF₂O- | 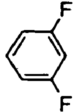 | OCF₃ | K97SmA115N 134 I; |
| | | | | | | | Clp. = 110 |
| | | | | | | | Δε = 34 |
| | | | | | | | Δn = 0,093 |
| 11 | CH₃ | - | 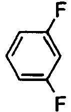 | -CF₂O- | 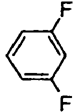 | CF₃ | |
| 12 | C₂H₅ | - | 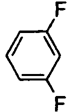 | -CF₂O- | 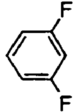 | CF₃ | |
| 13 | C₃H₇ | - | 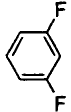 | -CF₂O- | 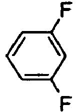 | CF₃ | K 111 N (99) I; |
| | | | | | | | Clp. = 81 |
| | | | | | | | Δε = 45 |
| | | | | | | | Δn = 0,098 |
| 14 | C₄H₉ | - | 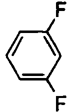 | -CF₂O- | 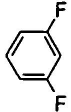 | CF₃ | |
| 15 | C₅H₁₁ | - | 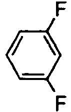 | -CF₂O- | 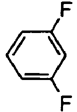 | CF₃ | |
| 16 | CH₃ | - | 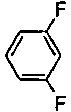 | -CF₂O- | 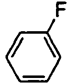 | F | |
| 17 | C₂H₅ | - | 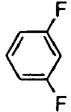 | -CF₂O- | 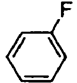 | F | |
| 18 | C₃H₇ | - | 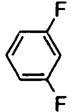 | -CF₂O- | 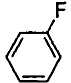 | F | |
| 19 | C₄H₉ | - | 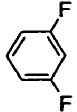 | -CF₂O- | 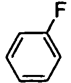 | F | |
| 20 | C₅H₁₁ | - | 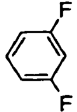 | -CF₂O- | 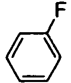 | F | |
| 21 | CH₃ | - | 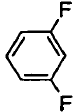 | -CF₂O- | 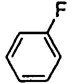 | OCF₃ | |
| 22 | C₂H₅ | - | 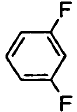 | -CF₂O- | 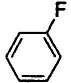 | OCF₃ | K 95 SmA 105 N 124 I |
| | | | | | | | Clp. = 105 |
| | | | | | | | Δε = 30 |
| | | | | | | | Δn = 0,096 |
| 23 | C₃H₇ | - | 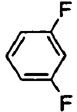 | -CF₂O- | 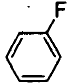 | OCF₃ | K 91 SmA130N 147 I |
| | | | | | | | Clp. = 124 |
| | | | | | | | Δε = 28 |
| | | | | | | | Δn = 0,105 |
| 24 | C₄H₉ | - | 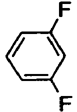 | -CF₂O- | 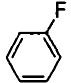 | OCF₃ | K90SmA136N 145 |
| | | | | | | | Clp. = 122 |
| | | | | | | | Δε = 27 |
| | | | | | | | Δn = 0,100 |
| | | | | | | | γ₁ = 543 |
| 25 | C₅H₁₁ | - | 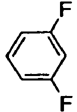 | -CF₂O- | 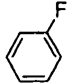 | OCF₃ | K 86 SmA 141 N 148 I |
| | | | | | | | Clp. = 127 |
| | | | | | | | Δε = 26 |
| | | | | | | | Δn = 0,103 |
| | | | | | | | γ₁ = 633 |
| 26 | CH₃ | - | 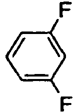 | -CF₂O- | 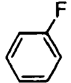 | CF₃ | |
| 27 | C₂H₅ | - | 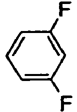 | -CF₂O- | 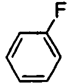 | CF₃ | |
| 28 | C₃H₇ | - | 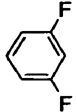 | -CF₂O- | 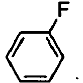 | CF₃ | |
| 29 | C₄H₉ | - | 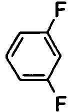 | -CF₂O- | 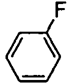 | CF₃ | |
| 30 | C₅H₁₁ | - | 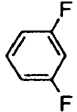 | -CF₂O- | 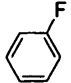 | CF₃ | |
| 31 | CH₃ | - | 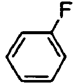 | -CF₂O- | 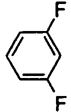 | F | |
| 32 | C₂H₅ | - | 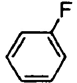 | -CF₂O- | 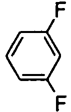 | F | |
| 33 | C₃H₇ | - | 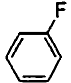 | -CF₂O- | 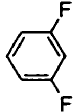 | F | K 68 SmA(A) 84 SmA 112 N 145 I; |
| | | | | | | | Clp. = 128 |
| | | | | | | | Δε = 29 |
| | | | | | | | Δn = 0,107 |
| 34 | C₄H₉ | - | 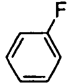 | -CF₂O- | 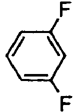 | F | |
| 35 | C₅H₁₁ | - | 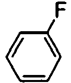 | -CF₂O- | 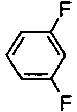 | F | |
| 36 | CH₃ | - | 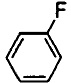 | -CF₂O- | 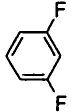 | OCF₃ | |
| 37 | C₂H₅ | - | 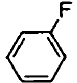 | -CF₂O- | 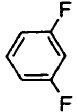 | OCF₃ | |
| 38 | C₃H₇ | - | 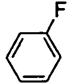 | -CF₂O- | 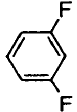 | OCF₃ | |
| 39 | C₄H₉ | - | 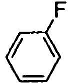 | -CF₂O- | 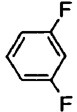 | OCF₃ | |
| 40 | C₅H₁₁ | - | 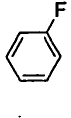 | -CF₂O- | 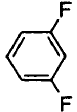 | OCF₃ | |
| 41 | CH₃ | - | 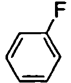 | -CF₂O- | 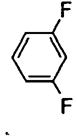 | CF₃ | |
| 42 | C₂H₅ | - | 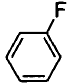 | -CF₂O- | 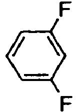 | CF₃ | |
| 43 | C₃H₇ | - | 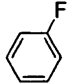 | -CF₂O- | 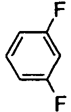 | CF₃ | |
| 44 | C₄H₉ | - | 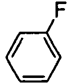 | -CF₂O- | 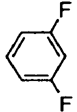 | CF₃ | |
| 45 | C₅H₁₁ | - | 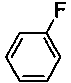 | -CF₂O- | 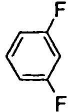 | CF₃ | |
| 46 | CH₃ | - | 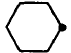 | -CF₂O- | 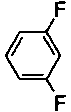 | F | |
| 47 | C₂H₅ | - | 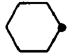 | -CF₂O- | 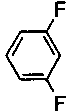 | F | |
| 48 | C₃H₇ | - | 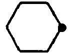 | -CF₂O- | 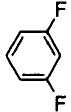 | F | K 88 SmB 175 N 207 I |
| | | | | | | | Clp. = 180 |
| | | | | | | | Δε= 24 |
| | | | | | | | Δn = 0,085 |
| | | | | | | | γ₁ = 813 |
| 49 | C₄H₉ | - | 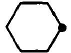 | -CF₂O- | 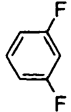 | F | |
| 50 | C₅H₁₁ | - | 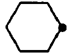 | -CF₂O- | 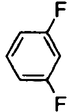 | F | |
| 51 | CH₃ | - | 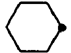 | -CF₂O- | 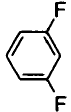 | OCF₃ | |
| 52 | C₂H₅ | - | 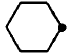 | -CF₂O- | 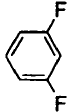 | OCF₃ | |
| 53 | C₃H₇ | - | 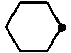 | -CF₂O- | 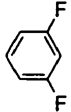 | OCF₃ | |
| 54 | C₄H₉ | - | 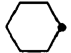 | -CF₂O- | 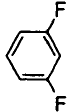 | OCF₃ | |
| 55 | C₅H₁₁ | - | 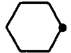 | -CF₂O- | 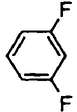 | OCF₃ | |
| 56 | CH₃ | - | 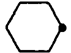 | -CF₂O- | 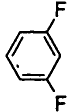 | CF₃ | |
| 57 | C₂H₅ | - | 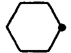 | -CF₂O- | 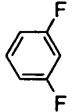 | CF₃ | |
| 58 | C₃H₇ | - | 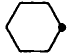 | -CF₂O- | 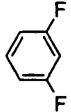 | CF₃ | |
| 59 | C₄H₉ | - | 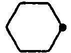 | -CF₂O- | 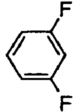 | CF₃ | |
| 60 | C₅H₁₁ | - | 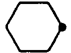 | -CF₂O- | 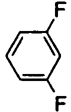 | CF₃ | |
| 61 | CH₃ | - | 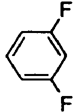 | - | 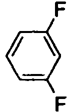 | F | |
| 62 | C₂H₅ | - | 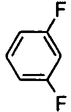 | - | 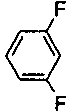 | F | |
| 63 | C₃H₇ | - | 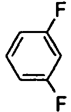 | - | 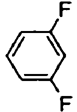 | F | K 103 N 148 I |
| | | | | | | | Clp. = 126 |
| | | | | | | | Δε=34 |
| | | | | | | | Δn = 0,124 |
| 64 | C₄H₉ | - | 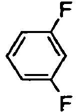 | - | 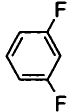 | F | |
| 65 | C₅H₁₁ | - | 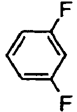 | - | 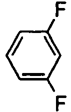 | F | |
| 66 | CH₃ | - | 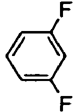 | - | 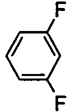 | OCF₃ | |
| 67 | C₂H₅ | - | 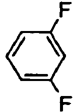 | - | 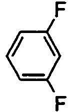 | OCF₃ | |
| 68 | C₃H₇ | - | 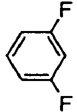 | - | 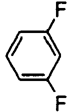 | OCF₃ | |
| 69 | C₄H₉ | - | 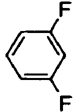 | - | 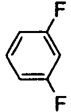 | OCF₃ | |
| 70 | C₅H₁₁ | - | 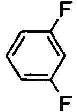 | - | 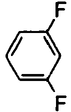 | OCF₃ | |
| 71 | CH₃ | - | 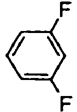 | - | 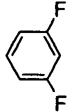 | CF₃ | |
| 72 | C₂H₅ | - | 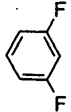 | - | 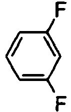 | CF₃ | |
| 73 | C₃H₇ | - | 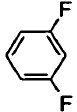 | - | 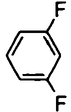 | CF₃ | |
| 74 | C₄H₉ | - | 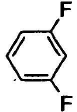 | - | 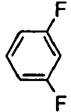 | CF₃ | |
| 75 | C₅H₁₁ | - | 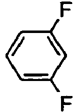 | - | 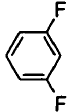 | CF₃ | |
| 76 | CH₃ | - | 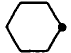 | - | 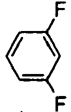 | F | |
| 77 | C₂H₅ | - | 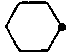 | - | 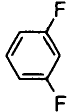 | F | |
| 78 | C₃H₇ | - | 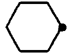 | - | 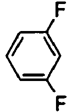 | F | K132SmB161 N 211 I; |
| | | | | | | | Clp. = 173 |
| | | | | | | | Δε = 22 |
| | | | | | | | Δn = 0,091 |
| 79 | C₄H₉ | - | 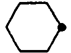 | - | 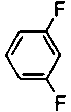 | F | |
| 80 | C₅H₁₁ | - | 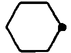 | - | 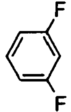 | F | |
| 81 | CH₃ | - | 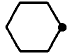 | - | 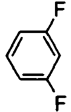 | OCF₃ | |
| 82 | C₂H₅ | - | 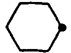 | - | 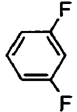 | OCF₃ | |
| 83 | C₃H₇ | - | 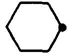 | - | 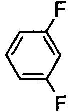 | OCF₃ | |
| 84 | C₄H₉ | - | 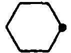 | - | 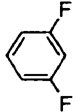 | OCF₃ | |
| 85 | C₅H₁₁ | - | 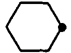 | - | 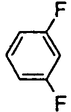 | OCF₃ | |
| 86 | CH₃ | - | 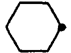 | - | 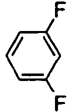 | CF₃ | |
| 87 | C₂H₅ | - | 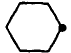 | - | 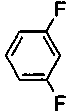 | CF₃ | |
| 88 | C₃H₇ | - | 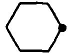 | - | 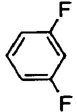 | CF₃ | |
| 89 | C₄H₉ | - | 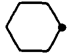 | - | 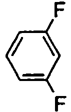 | CF₃ | |
| 90 | C₅H₁₁ | - | 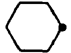 | - | 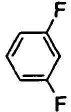 | CF₃ | |
| 91 | CH₃ | - | 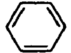 | - | 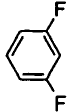 | F | |
| 92 | C₂H₅ | - | 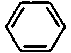 | - | 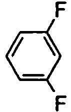 | F | |
| 93 | C₃H₇ | - | 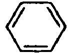 | - | 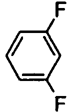 | F | K 101 SmA(A) 104 SmA 197 N 223I |
| | | | | | | | Clp. = 201 |
| | | | | | | | Δε = 26 |
| | | | | | | | Δn = 0,150 |
| 94 | C₄H₉ | - | 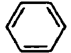 | - | 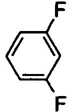 | F | |
| 95 | C₅H₁₁ | - | 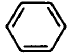 | - | 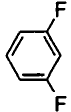 | F | |
| 96 | CH₃ | - | 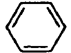 | - | 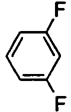 | OCF₃ | |
| 97 | C₂H₅ | - | 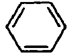 | - | 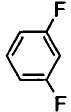 | OCF₃ | |
| 98 | C₃H₇ | - | 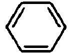 | - | 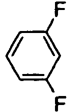 | OCF₃ | |
| 99 | C₄H₉ | - | 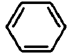 | - | 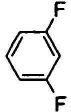 | OCF₃ | |
| 100 | C₅H₁₁ | - | 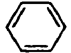 | - | 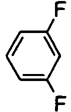 | OCF₃ | |
| 101 | CH₃ | - | 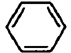 | - | 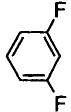 | CF₃ | |
| 102 | C₂H₅ | - | 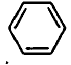 | - | 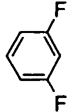 | CF₃ | |
| 103 | C₃H₇ | - | 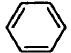 | - | 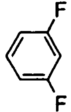 | CF₃ | |
| 104 | C₄H₉ | - | 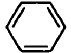 | - | 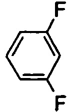 | CF₃ | |
| 105 | C₅H₁₁ | - | 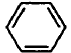 | - | 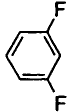 | CF₃ | |
| 106 | CH₃ | 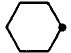 | 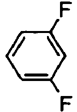 | -CF₂O- | 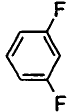 | F | |
| 107 | C₂H₅ | 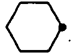 | 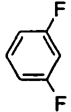 | -CF₂O- | 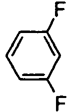 | F | |
| 108 | C₃H₇ | 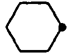 | 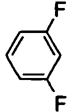 | -CF₂- | 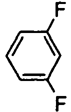 | F | K 95 N 251 I |
| | | | | | | | Clp. = 219 |
| | | | | | | | Δε = 30 |
| | | | | | | | Δn = 0,120 |
| 109 | C₄H₉ | 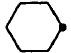 | 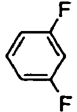 | -CF₂O- | 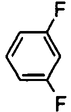 | F | |
| 110 | C₅H₁₁ | 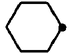 | 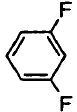 | -CF₂O- | 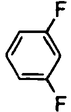 | F | |
| 111 | CH₃ | 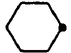 | - | - | 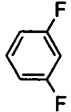 | F | |
| 112 | C₂H₅ | 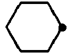 | - | - | 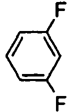 | F | |
| 113 | C₃H₇ | 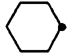 | - | - | 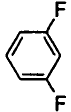 | F | K118N206 I |
| | | | | | | | Clp. = 190 |
| | | | | | | | Δε =21 |
| | | | | | | | Δn = 0,089 |
| | | | | | | | γ1 = 1330 |
| 114 | C₄H₉ | 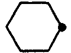 | - | - | 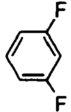 | F | K 107 N 2101 |
| | | | | | | | Clp. = 195 |
| | | | | | | | Δε = 22 |
| | | | | | | | Δn = 0,101 |
| | | | | | | | γ1 = 1457 |
| 115 | C₅H₁₁ | 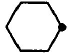 | - | - | 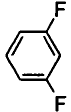 | F | |

## Patentansprüche

1. Verbindungen der allgemeinen Formel I 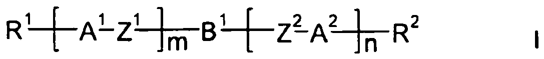 worin
B¹ 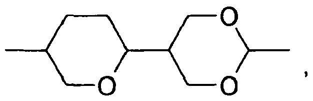
R¹, R² H, Halogen, CN, SCN, NCS, SF₅, einen linearen oder verzweigten, gegebenenfalls chiralen, unsubstituierten, einen einfach durch CN oder CF₃ substituierten oder einen ein- oder mehrfach durch Halogen substituierten Alkylrest mit 1 bis 15 C-Atomen bedeutet, worin auch eine oder mehrere CH₂-Gruppen jeweils unabhängig voneinander durch -O-, -S-, -CO-, -CO-O-, -O-CO-, -CH=CH-, -CH=CF-, -CF=CF-, -C≡C-, -CH₂O- oder -CF₂O- so ersetzt sein können, dass Heteroatome nicht direkt miteinander verknüpft sind und unsymmetrische Gruppen in beiden Orientierungen vorliegen können,
A¹, A² jeweils unabhängig voneinander, gleich oder verschieden
a) trans-1,4-Cyclohexylen, worin auch eine oder mehrere nicht benachbarte CH₂-Gruppen durch -O- und/oder -S-ersetzt sein können,
b) 1,4-Phenylen, worin eine oder zwei CH-Gruppen durch N ersetzt sein können und worin auch ein oder mehrere H-Atome gegen Br, Cl, F, CN, Methyl, Methoxy oder eine ein- oder mehrfach fluorierte Methyl- oder Methoxygruppe ersetzt sein können,
c) ein Rest aus der Gruppe 1,4-Bicyclo(2,2,2)-octylen, Piperidin-1,4-diyl, Cylcobut-1,3-diyl, Spiro[3.3]heptan-2,6-diyl, 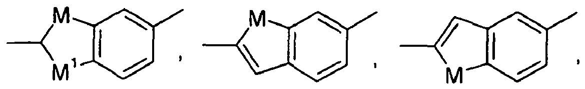 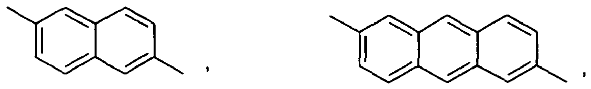 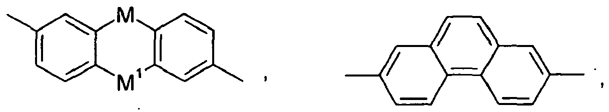 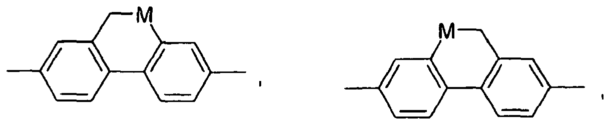 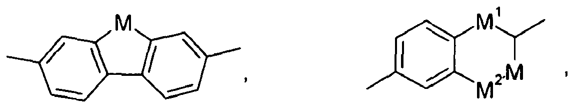 worin Wasserstoffatome ein oder mehrfach durch F, CN, SCN, SF₅, CH₂F, CHF₂ oder CF₃, OCH₂F, OCHF₂ oder OCF₃ substituiert sein können, eine oder mehrere Doppelbindungen durch Einfachbindungen ersetzt sein können, M, M¹ oder M² -O-, -S-, -CH₂-, -CHY- oder -CYY¹-, undY und Y¹ Cl, F, CN, OCF₃ oder CF₃ bedeuten, oder
d) 1,4-Cyclohexenylen,
Z¹, Z² jeweils unabhängig voneinander, gleich oder verschieden, eine Einfachbindung, -CH₂O-, -CO-O-, -CF₂O-, -CH₂CH₂CF₂O-, -CF₂CF₂-, -CH₂CF₂-, -CH₂CH₂-, -CH=CH-, -CH=CF-, -CF=CF- oder -C≡C-, wobei asymmetrische Brücken nach beiden Seiten orientiert sein können, und
n, m unabhängig voneinander 0, 1, 2 oder 3, wobei m + n ≥ 2 ist
bedeuten.

2. Verbindungen gemäß dem Anspruch 1, **dadurch gekennzeichnet, dass**
n 1, 2 oder 3 bedeutet, und mindestens eine der Gruppen Z² -CF₂O-, -CO-O-, -CF=CF-, -CH₂O- oder -CF₂CF₂-
bedeuten.

3. Verbindungen gemäß Anspruch 1 oder 2 der Unterformel IA 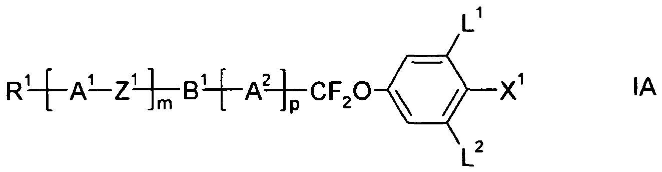 worin
p 0, 1 oder 2,
m + p 0, 1, oder 2, und
L¹, L² unabhängig voneinander, H, F, Cl, CN oder CF₃, und
X¹ H, Halogen, CN, SCN, NCS, SF₅, einen linearen oder verzweigten, einfach durch CN oder CF₃ oder einen mindestens einfach durch Halogen substituierten Alkylrest mit 1 bis 8 C-Atomen bedeutet, worin auch eine oder mehrere CH₂-Gruppen jeweils unabhängig voneinander durch -O-, -CF=CF- oder -C≡C- so ersetzt sein können, dass Heteroatome nicht direkt miteinander verknüpft sind,
bedeuten.

4. Verbindungen gemäß dem vorangehenden Anspruch der Unterformel IB 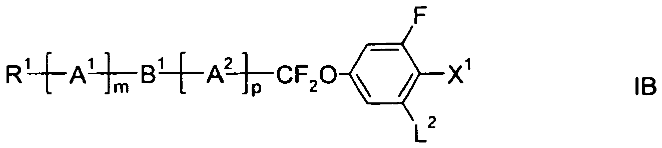 worin
L² H oder F,
X¹ F oder OCF₃, CF₃, CN, SCN, NCS, SF₅,
A¹ ein 1,4-Cyclohexandiyl,
A² eine Gruppe der Formeln 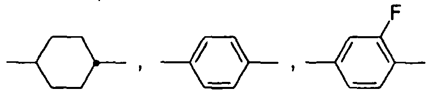 oder 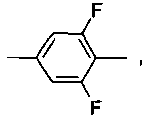
m 0 oder 1,
p 0 der 1, und
m+p 0 oder 1,
bedeuten.

5. Verbindungen gemäß mindestens einem der vorangehenden Ansprüche, **dadurch gekennzeichnet, dass**
R¹ einen linearen Alkyl- bzw. Alkoxyrest mit 1 bis 12 C-Atomen oder
einen linearen Alkenyl- bzw. Alkenyloxyrest mit 2 bis 12 C-Atomen bedeutet.

6. Verbindungen gemäß Anspruch 1 oder 2, **dadurch gekennzeichnet, dass**
Z¹ und Z² unabhängig voneinander -CF₂O-, -CF₂CF₂-, -CF=CF- oder eine Einfachbindung bedeuten.

7. Verwendung einer oder mehrerer Verbindungen gemäß mindestens einem der vorangehenden Ansprüche als Komponente(n) in flüssigkristallinen Medien.

8. Flüssigkristallines Medium mit mindestens zwei flüssigkristallinen Komponenten, **dadurch gekennzeichnet, dass** es mindestens ein Pyran-Dioxan-Derivat gemäß mindestens einem der Ansprüche 1 bis 6 enthält.

9. Flüssigkristall-Anzeigeelement, **dadurch gekennzeichnet, dass** es ein flüssigkristallines Medium gemäß Anspruch 8 enthält.

10. Elektrooptisches Anzeigeelement, **dadurch gekennzeichnet, dass** es als Dielektrikum ein flüssigkristallines Medium gemäß Anspruch 8 enthält.

## Claims

1. Compounds of the general formula I 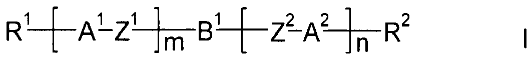 in which
B¹ denotes 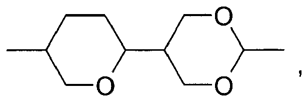
R¹, R² denote H, halogen, CN, SCN, NCS, SF₅, a linear or branched, optionally chiral alkyl radical having 1 to 15 C atoms which is unsubstituted, monosubstituted by CN or CF₃ or mono- or polysubstituted by halogen and in which one or more CH₂ groups may each be replaced, independently of one another, by -O-, -S-, -CO-, -CO-O-, -O-CO-, -CH=CH-, -CH=CF-, -CF=CF-, -C≡C-, -CH₂O- or -CF₂O- in such a way that heteroatoms are not linked directly to one another and asymmetrical groups may be present in both orientations,
A¹, A² each, independently of one another, identically or differently, denote
a) trans-1,4-cyclohexylene, in which, in addition, one or more non-adjacent CH₂ groups may be replaced by -O- and/or -S-,
b) 1,4-phenylene, in which one or two CH groups may be replaced by N and in which, in addition, one or more H atoms may be replaced by Br, Cl, F, CN, methyl, methoxy or a mono- or polyfluorinated methyl or methoxy group,
c) a radical from the group 1,4-bicyclo[2.2.2]octylene, piperidine-1,4-diyl, cyclobutane-1,3-diyl, spiro[3.3]-heptane-2,6-diyl, 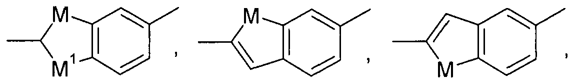 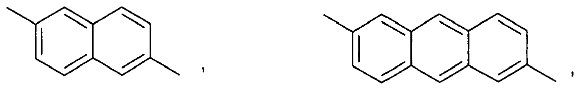 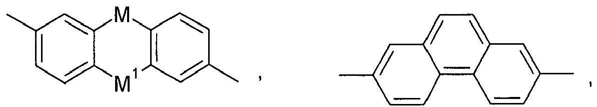 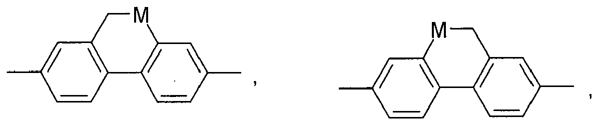 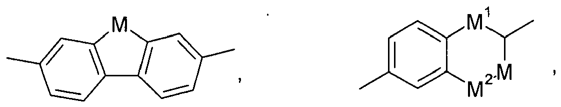 in which hydrogen atoms may be mono- or polysubstituted by F, CN, SCN, SF₅, CH₂F, CHF₂ or CF₃, OCH₂F, OCHF₂ or OCF₃,
one or more double bonds may be replaced by single bonds,
M, M¹ or M² denotes -O-, -S-, -CH₂-, -CHY- or -CYY¹-, and
Y and Y¹ denote Cl, F, CN, OCF₃ or CF₃,
or
d) 1,4-cyclohexenylene,
Z¹, Z² each, independently of one another, identically or differently, denote
a single bond, -CH₂O-, -CO-O-, -CF₂O-, -CH₂CH₂CF₂O-, -CF₂CF₂-, -CH₂CF₂-, -CH₂CH₂-, -CH=CH-, -CH=CF-, -CF=CF- or -C≡C-, where asymmetrical bridges may be oriented to both sides, and
n, m, independently of one another, denote 0, 1, 2 or 3,
where m + n ≥ 2.

2. Compounds according to Claim 1, **characterised in that**
n denotes 1, 2 or 3, and at least one of the groups Z² denotes -CF₂O-, -CO-O-, -CF=CF-, -CH₂O- or -CF₂CF₂-.

3. Compounds according to Claim 1 or 2, of the sub-formula IA 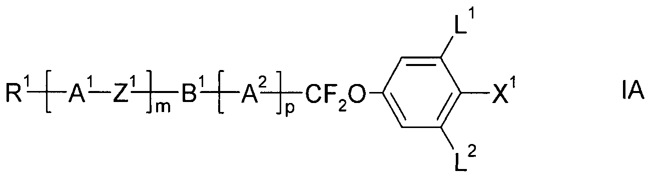 in which
p denotes 0, 1 or 2,
m + p denotes 0, 1 or 2,
L¹, L², independently of one another, denote H, F, Cl, CN or CF₃, and
X¹ denotes H, halogen, CN, SCN, NCS, SF₅, a linear or branched alkyl radical having 1 to 8 C atoms which is monosubstituted by CN or CF₃ or at least monosubstituted by halogen and in which one or more CH₂ groups may each be replaced, independently of one another, by -O-, -CF=CF- or -C≡C- in such a way that heteroatoms are not linked directly to one another.

4. Compounds according to the preceding claim, of the sub-formula IB 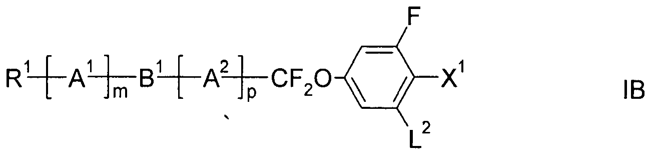 in which
L² denotes H or F,
X¹ denotes F or OCF₃, CF₃, CN, SCN, NCS, SF₅,
A¹ denotes a 1,4-cyclohexanediyl,
A² denotes a group of the formula 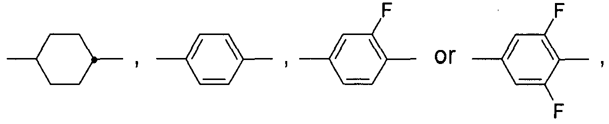
m denotes 0 or 1,
p denotes 0 or 1, and
m + p denotes 0 or 1.

5. Compounds according to at least one of the preceding claims, **characterised in that**
R¹ denotes a linear alkyl or alkoxy radical having 1 to 12 C atoms or a linear alkenyl or alkenyloxy radical having 2 to 12 C atoms.

6. Compounds according to Claim 1 or 2, **characterised in that**
Z¹ and Z², independently of one another, denote -CF₂O-, -CF₂CF₂-, -CF=CF- or a single bond.

7. Use of one or more compounds according to at least one of the preceding claims as component(s) in liquid-crystalline media.

8. Liquid-crystalline medium having at least two liquid-crystalline components, **characterised in that** it comprises at least one pyran-dioxane derivative according to at least one of Claims 1 to 6.

9. Liquid-crystal display element, **characterised in that** it contains a liquid-crystalline medium according to Claim 8.

10. Electro-optical display element, **characterised in that** it contains, as dielectric, a liquid-crystalline medium according to Claim 8.

## Revendications

1. Composés de la formule générale I 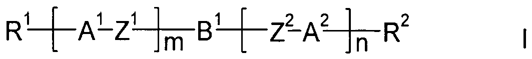 dans laquelle
B¹ représente 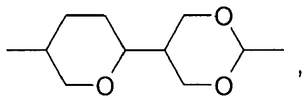
R¹, R² représente H, halogène, CN, SCN, NCS, SF₅, un radical alkyle en option chiral, linéaire ou ramifié, comportant de 1 à 15 atomes de C, lequel est non substitué, monosubstitué par CN ou CF₃ ou mono- ou polysubstitué par halogène et où un ou plusieurs groupes CH₂ peuvent chacun être remplacés, indépendamment les uns des autres, par -O-, -S-, -CO-, -CO-O-, -O-CO-, -CH=CH-, -CH=CF-, -CF=CF-, -C≡C-, -CH₂O- ou -CF₂O- de telle sorte que des hétéroatomes ne soient pas liés directement les uns aux autres et que des groupes asymétriques puissent être présents suivant les deux orientations,
A¹, A² représentent, chacun indépendamment de l'autre, de manière identique ou différente
a) trans-1,4-cyclohexylène, où, en outre, un ou plusieurs groupes CH₂ non adjacents peut/peuvent être remplacé(s) par -O- et/ou -S-,
b) 1,4-phénylène, où un ou deux groupes CH peut/peuvent être remplacé(s) par N et où, en outre, un ou plusieurs atomes de H peut/peuvent être remplacé(s) par Br, Cl, F, CN, méthyle, méthoxy ou un groupe méthyle ou méthoxy mono- ou polyfluoré,
c) un radical pris parmi le groupe 1,4-bicyclo[2.2.2]octylène, pipéridine-1,4-diyle, cyclobutane-1,3-diyle, spiro[3.3]heptane-2,6-diyle, 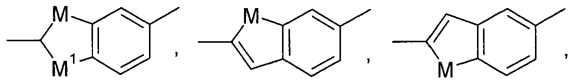 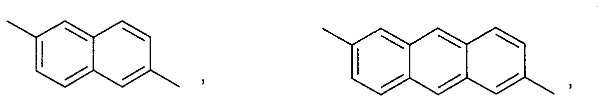 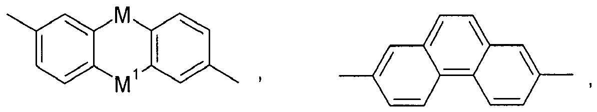 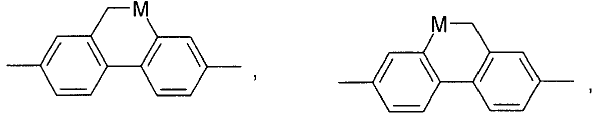 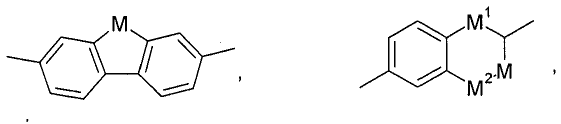 où des atomes d'hydrogène peuvent être mono- ou polysubstitués par F, CN, SCN, SF₅, CH₂F, CHF₂ ou CF₃, OCH₂F, OCHF₂ ou OCF₃, une ou plusieurs liaisons doubles peut/peuvent être remplacée(s) par des liaisons simples,
M, M¹ ou M² représente -O-, -S-, -CH₂-, -CHY- ou -CYY¹- et
Y et Y¹ représente Cl, F, CN, OCF₃ ou CF₃,
ou
d) 1,4-cyclohexénylène,
Z¹, Z² représentent, chacun indépendamment de l'autre, de manière identique ou différente, une liaison simple, -CH₂O-, -CO-O-, -CF₂O-, -CH₂CH₂CF₂O-, -CF₂CF₂-, -CH₂CF₂-, -CH₂CH₂-, -CH=CH-, -CH=CF-, -CF=CF- ou -C≡C-, où des ponts asymétriques peuvent être orientés sur les deux côtés, et
n, m, représentent, indépendamment l'un de l'autre, 0, 1, 2 ou 3, où m + n ≥ 2.

2. Composés selon la revendication 1, **caractérisés en ce que**
n représente 1, 2 ou 3, et au moins l'un des groupes Z² représente -CF₂O-, -CO-O-, -CF=CF-, -CH₂O- ou -CF₂CF₂-.

3. Composés selon la revendication 1 ou 2, de la sous-formule IA 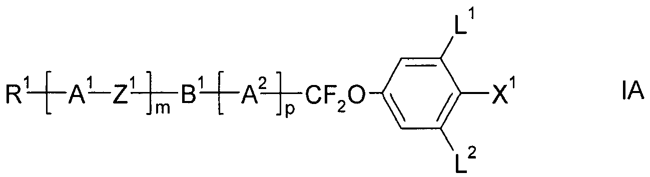 dans laquelle
p représente 0, 1 ou 2,
m + p représente 0, 1 ou 2,
L¹, L², représentent, indépendamment l'un de l'autre, H, F, Cl, CN ou CF₃, et
X¹ représente H, halogène, CN, SCN, NCS, SF₅, un radical alkyle linéaire ou ramifié comportant de 1 à 8 atomes de C, lequel est monosubstitué par CN ou CF₃ ou au moins monosubstitué par halogène et où un ou plusieurs groupes CH₂ peuvent chacun être remplacé(s), indépendamment les uns des autres, par -O-, -CF=CF- ou -C≡C- de telle sorte que des hétéroatomes ne soient pas liés directement les uns aux autres.

4. Composés selon la revendication précédente, de la sous-formule IB 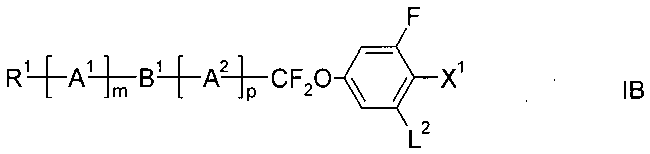 dans laquelle
L² représente H ou F,
X¹ représente F ou OCF₃, CF₃, CN, SCN, NCS, SF₅,
A¹ représente un 1,4-cyclohexanediyle,
A² représente un groupe de la formule 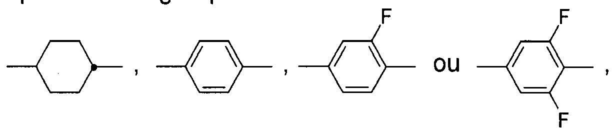
m représente 0 ou 1,
p représente 0 ou 1, et
m + p représente 0 ou 1.

5. Composés selon au moins l'une des revendications précédentes,
**caractérisés en ce que**
R¹ représente un radical alkyle ou alcoxy linéaire comportant de 1 à 12 atomes de C ou un radical alkényle ou alkényloxy linéaire comportant de 2 à 12 atomes de C.

6. Composés selon la revendication 1 ou 2, **caractérisés en ce que** Z¹ et Z² représentent, indépendamment l'un de l'autre, -CF₂O-, -CF₂CF₂-, -CF=CF- ou une liaison simple.

7. Utilisation d'un ou de plusieurs composés selon au moins l'une des revendications précédentes en tant que composant(s) dans des milieux cristallins liquides.

8. Milieu cristallin liquide comportant au moins deux composants cristallins liquides, **caractérisé en ce qu'**il comprend au moins un dérivé de pyrane-dioxane selon au moins l'une des revendications 1 à 6.

9. Elément d'affichage à cristaux liquides, **caractérisé en ce qu'**il contient un milieu cristallin liquide selon la revendication 8.

10. Elément d'affichage électro-optique, **caractérisé en ce qu'**il contient, en tant que diélectrique, un milieu cristallin liquide selon la revendication 8.
